# EUROPEAN PATENT SPECIFICATION

(11) **EP 2 214 681 B1**
(45) Date of publication and mention of the grant of the patent: **20.10.2021**
(21) Application number: 08857760.6
(22) Date of filing: 03.12.2008
(51) Int. Cl.: A61K 31/704, A61K 36/28, A61P 25/00, A61P 43/00, A23K 20/163, A23K 20/10, A23K 50/40, A23L 27/30, A23L 33/105

(54) **NOVEL NUTRACEUTICAL COMPOSITIONS CONTAINING STEVIOL AND USES THEREOF**
NEUE FUNKTIONELLE LEBENSMITTEL ENTHALTEND STEVIOL UND DEREN VERWENDUNGEN
NOUVELLES COMPOSITIONS NUTRACEUTIQUES CONTENANT DU STEVIOL ET LEURS UTILISATIONS

(30) Priority: 03.12.2007 EP 07023334
(43) Date of publication of application: 11.08.2010
(73) Proprietor: DSM IP Assets B.V., 6411 TE Heerlen (NL)
(72) Inventor: FOWLER, Ann, CH-4310 Rheinfelden (CH); GORALCZYK, Regina, 79639 Grenzach-Wyhlen (DE); KILPERT, Claus, 68305 Mannheim (DE); MAYNE-MECHAN, Annis, Olivia, CH-4313 Möhlin (CH); MOHAJERI, Hasan, CH-8132 Egg b. Zürich (CH); MUSSLER, Bernd, 77933 Lahr (DE); WYSS, Adrian, CH-4052 Basel (CH)
(74) Representative: Rabanus, Birgit
(86) International application number: PCT/EP2008/010231
(87) International publication number: WO 2009/071277

(56) References cited:
- RU-C1- 2 160 310
- US-A1- 2007 116 840
- US-A1- 2007 224 292
- DATABASE WPI Week 199545 Thomson Scientific, London, GB; AN 1995-345071 XP002516539 & CN 1 094 892 A (LIU J) 16 November 1994 (1994-11-16)
- MELIS M S ED - SCHEER RAINER ET AL: "Effects of steviol on renal function and mean arterial pressure in rats", PHYTOMEDI, ELSEVIER, AMSTERDAM, NL, vol. 3, no. 4, 1 January 1997 (1997-01-01) , pages 349-352, XP009151840, ISSN: 0944-7113

## Description

### FIELD OF THE INVENTION

The present invention relates to the use of a nutraceutical composition or food additive comprising steviol as an active ingredient to improve cognitive functions, such as learning, memory and alertness, as well as relieving psychosocial pressure.

### BACKGROUND OF THE INVENTION

Memory, learning and alertness rely on neuronal circuits in the midbrain, especially in the hippocampus where information is processed and memory is consolidated. Mental performance and learning are dependent on synaptic plasticity; i.e. strengthening of neuronal connections by the recruitment of new receptors, formation of new synapses and eventually the generation of new neuronal connections.

The formation of (long-term) memory and the efficient functioning of the brain depend on synthesis of new proteins for the reinforcement of communicative strength between neurones. The production of new proteins devoted to synapse reinforcement is triggered by chemical and electrical signals within neurones.

Long term potentiation (LTP) is the term used to describe the long-lasting enhancement of synaptic transmission (hours *in vitro,* days or weeks *in vivo)* which occurs at particular synapses within the central nervous system (CNS) following a short, conditioning, burst of presynaptic electrical stimulation (approximately 100 Hz for 1 second). This phenomenon is widely considered to be one of the major mechanisms by which memories are formed and stored in the brain. LTP has been observed both *in vitro* and in living animals. Under experimental conditions, applying a series of short, high-frequency electrical stimuli to a synapse can potentiate the strength of the chemical synapse for minutes to hours. Most importantly, LTP contributes to synaptic plasticity in living animals, providing the foundation for a highly adaptable nervous system.

Two different receptor types are primarily involved in the process of LTP, namely the N-methyl-D-aspartate (NMDA) receptor complex and the α-amino-3-hydroxy-5-methyl-4-isoxazole propionic acid (AMPA) receptor. During LTP, the major excitatory neurotransmitter, glutamate, is released from the presynaptic neurone, binds to and activates the AMPA receptor on the postsynaptic membrane, leading to its depolarisation. At resting membrane potentials, the NMDA receptor channel is blocked by magnesium ions, but depolarisation of the postsynaptic membrane removes this block, enabling NMDA receptor activation and subsequent entry of calcium into the cell. This rise in intracellular calcium is believed to activate protein kinases, leading to gene transcription and the construction of reinforcing proteins (Niehoff (2005), The Language of Life: How Cells Communicate in Health and Disease, 210-223) and resulting in enhanced sensitivity of the AMPA receptor, thus further facilitating neurotransmission and maintenance of LTP.

NMDA receptors are composed of assemblies of NR1- and NR2- subunits; the glutamate binding domain is formed at the junction of these subunits. In addition to glutamate, the NMDA receptor requires a co-agonist, glycine, in order to modulate receptor function. The glycine binding site is found on the NR1 subunit, while the NR2 subunit possesses a binding site for polyamines, regulatory molecules that modulate the functioning of the NMDA receptor.

The amino acid glycine is thus known to act as a positive allosteric modulator and obligatory co-agonist with glutamate at the NMDA receptor complex (Danysz and Parsons 1998 Pharmacol. Rev., 50(4):597-664). Glycine transporters (GlyT) play an important role in the termination of postsynaptic glycinergic actions and maintenance of low extracellular glycine concentrations by reuptake of glycine into presynaptic nerve terminals or glial cells. The termination of the action of glycine is therefore largely mediated by rapid reuptake. Two glycine transporters, GlyT1 and GlyT2, are known and are characterised by 12 putative transmembrane regions, while three variants of GlyT1 (GlyT1a, b, and c) encoded from the same gene have been identified (Borowsky and Hoffman 1998 J. Biol. Chem., 273(44):29077-29085).

GlyT1 is the only sodium chloride-dependent glycine transporter in the forebrain, where it is co-expressed with the NMDA receptor. At this site, GlyT1 is thought to be responsible for controlling extracellular levels of glycine at the synapse (Lopez-Corcuera et al 2001 Mol. Membr. Biol., 18(1): 13-20), resulting in modulation of NMDA receptor function.

Indeed, in the presence of the selective GlyT1 antagonist *N*-[3-(4'-fluorophenyl)-3-(4'-phenylphenoxy)] propylsarcosine (NFPS), enhanced NMDA receptor responses in CA1 pyramidal cells were observed upon Schaffer collateral stimulation in both mouse and rat hippocampal slice preparations (Bergeron, et al 1998, Proc. Natl. Acad. Sci. USA, 95(26): 15730-15734). *In vivo,* systemic administration of NFPS increased LTP in the dentate gyrus and enhanced prepulse inhibition of the acoustic startle response in adult mice, indicating that inhibition of GlyT1 affects NMDA receptor function in a behaviourally-relevant way (Kinney, et al 2003, J. Neurosci., 23(20):7586-7591).

Such data highlight the potential usefulness of compounds which can enhance NMDA receptor synaptic function by elevating extracellular levels of glycine in the local microenvironment of synaptic NMDA receptors, for the prevention of psychotic syndromes and for maintaining or boosting physiological cognitive functions, such as memory and learning, in normal individuals.

There is an increasing interest in the development of compounds, as well as nutraceutical compositions, that may be used to improve learning, memory and alertness, in both elderly and young people, individuals who need especially high memory and attention in their daily work, including students, construction workers, drivers, pilots, physicians, salespeople, executives, housewives, "high performance professionals" and people who are under mental or daily stress as well as persons who are prone to psychiatric instability, such as schizophrenia.

Thus, a compound or nutraceutical composition which enhances NMDA receptor function and enables improvements in learning, memory and alertness would be highly desirable. *Stevia* extracts have been added to beverages/food compositions in the past, (for example see Japanese Kokai 2005-278467 to Nippon Paper Chemical Co., Ltd), but the *Stevia* extract functioned as a sweetener.

US2007/224292 (Brunner et al) discloses a beverage which contains: caffeine, chromium polynicotinate, niacin, calcium, potassium, magnesium, sodium and Vitamin C. Stevia power may also be added.

Melis, M.S. 1996 Phytomedicine 3(4):349-352; XP009151840 discloses that a steviol infusion affects salt and water transport in renal tubules in rats, and may have use as a hypotensive agent.

### DETAILED DESCRIPTION OF THE INVENTION

It has been found, in accordance with this invention that compounds of Formulae I and II, below, are activators of hippocampal function, through their ability to induce LTP, and as such are useful as nutraceuticals and/or pharmaceuticals which enhance cognitive functions. These compounds can function either by inhibiting the glycine transporter, GlyT1, thus inhibiting reuptake of glycine, or by activation of another pathway which leads to LTP induction, or by both mechanisms. Thus, this invention relates to a method of enhancing cognitive functions by administering a cognitive-function enhancing amount of steviol to an animal (including humans).

Additionally, it has been found in accordance with this invention that steviol, induces LTP.

Another aspect of this invention is the use of steviol to enhance cognitive functions.

Therefore one aspect of the invention is a novel nutraceutical composition, comprising steviol.

Formula I: See Table 1 for details of R groups

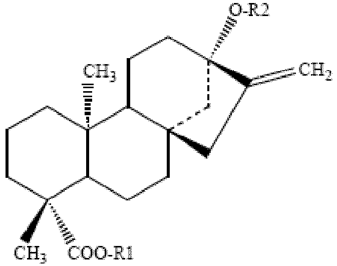

Table 1: Structures of steviol and related glycosides (Formula I). Glc, Xyl and Rha represent, respectively, glucose, xylose and rhamnose sugar moieties (Kuznesof (2007) Steviol glycosides - chemical and technical assessment, 68th JECFA meeting).

| **Compound name** | **R1** | **R2** |
|---|---|---|
| Steviol | H | H |
| Steviolbioside | H | β-Glc-β-Glc(2→1) |
| Stevioside | β-Glc | β-Glc-β-Glc(2→1) |
| Rebaudioside A | β-Glc | β-Glc-β-Glc(2→1) |
| | | \| |
| | | β-Glc(3→1) |
| Rebaudioside B | H | β-Glc-β-Glc(2→1) |
| | | \| |
| | | P-Glc(3→1) |
| Rebaudioside C | β-Glc | β-Glc-α-Rha(2→1) |
| (dulcoside B) | | \| |
| | | β-Glc(3→1) |
| Rebaudioside D | β-Glc-β-Glc(2→1) | β-Glc-β-Glc(2→1) |
| | | \| |
| | | β-Glc(3→1) |
| Rebaudioside E | β-Glc-β-Glc(2→1) | β-Glc-β-Glc(2→1) |
| Rebaudioside F | β-Glc | β-Glc-P-Xyl(2→1) |
| | | \| |
| | | β-Glc(3→1) |
| Rubusoside | β-Glc | β-Glc |
| dulcoside A | β-Glc | β-Glc-α-Rha(2→1) |

Formula II: Isosteviol (CAS number, 27975-19-5) is a decomposition product of steviol glycosides.

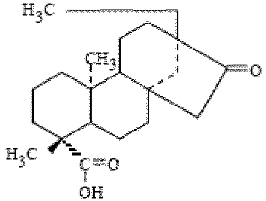

The compounds of Formula I and II can either be synthetically produced using known methods, be extracted from natural sources, such as plants, using known extraction procedures, or they may be used as a component of a plant extract, preferably a *Stevia* extract, which contains sufficient amounts of steviol and/or stevioside to be an effective enhancer of hippocampal function.

As previously described, the basis of memory, learning and mental stability is LTP, or the strengthening of neuronal connections, which occurs via activation of AMPA and NMDA receptors within the brain, particularly in the hippocampus. As glycine reuptake inhibitors, through their activity at GlyT1, *Stevia* extracts allow accumulation of glycine in the vicinity of the NMDA receptor, thus activating it and ultimately resulting in the induction of LTP, the main cellular mechanism involved in memory formation and consolidation.

Moreover, steviol, isosteviol and stevioside also induce activation of the same biochemical pathway (albeit at a different step than *Stevia* extract), leading to LTP induction, and are likewise beneficial in improving memory functions.

*Stevia* extract and its constituents can therefore activate hippocampal functions and improve memory formation and consolidation, as well as mental health. Thus, as used throughout the specification and claims, the term a "cognitive-function enhancing amount" means that the dosage is sufficient to activate hippocampal functions and can lead to improved cognitive function, which is explained further below.

### Conditions improved by this invention:

In the context of this invention "treatment" also encompasses co-treatment as well as prevention, lessening the symptoms associated with a particular condition, decreasing the time of onset of a particular condition, lessening the severity of a condition, and decreasing the likihood that an asymptomatic individual will show a condition in the future.

Throughout this specification and claims, the term "improved cognitive function" is meant to refer to the conditions of supporting and maintaining cognitive wellness and balance, such as:
- Enhanced learning, including:
   ∘ language processing
   ∘ problem solving
   ∘ intellectual functioning
- Ability to cope with psychosocial burdens
- Enhanced attention and concentration
- Enhanced memory and the capacity for remembering, especially short-term memory
- Enhanced mental alertness and mental vigilance, reduction of mental fatigue
- Stabilisation of mental status including:
   ∘ Relieving post-partum conditions
   ∘ Relieving psychological burden due to separation of partners, children, death of beloved people or due to marital problems
   ∘ Relieving problems associated with change of domicile or work, or similar events
   ∘ Relieving stressful conditions following a traffic accident or other negative social pressure.
- Stress relief, including:
   ∘ treatment, prevention and alleviation of symptoms related to work overload, exhaustion and/or burn out
   ∘ increased resistance or tolerance to stress
   ∘ favouring and facilitating relaxation in normal healthy individuals
- "Condition improvement", including:
   ∘ reducing irritability and tiredness
   ∘ Ability to cope with new situations
   ∘ reducing, preventing or alleviating physical and mental fatigue
   ∘ promoting good-quality sleep, that is to act against insomnia and sleep disorders and to increase energy in more general terms, in diseased or normal healthy individuals.

In a preferred aspect of the present invention the compositions may be used as nutritional supplements, particularly for people who may feel a need for enhanced cognitive function and / or psychosocial support. A non-exhaustive list of people who would benefit from enhanced cognitive function would include:
elderly people,
students or persons who are preparing for exams,
children who are engaged in a great deal of learning, i.e. infants, toddlers, pre-school children and school children,
construction workers, or those operating potentially dangerous machinery,
truck drivers, pilots, train drivers, or other transportation professionals,
air traffic controllers,
salespeople, executives, and other "high performance professionals"
police officers and military personnel,
housewives,
or for anyone exposed to high amounts of stress in their daily work or who needs especially high attention/concentration/high mental and psychological performance in their daily work, such as those participating in sports, chess players, golfers, professional performers (actors, musicians and the like).

To achieve these improvements, administration over several days (for example at least six or ten days) is recommended, and administration daily for several weeks is generally preferred.

Aside from applications for humans, the compositions of this invention have additional uses in the veterinary world. Animals which can benefit from enhanced cognitive function include those animals which are subject to stressful conditions. Such conditions occur, for example, after capture or transport or may be due to housing conditions (such as change of domicile or owner), when the animals develop analogous disorders and are distressed or aggressive, or display stereotypic behaviour, or anxiety and obsessive-compulsive behaviour. Animals which are subject to stress would also include those which are racing animals (e.g. dogs, horses, camels), or used in various sports, performing animals (such as circus animals and those appearing on stage, television or in the movies) and horses which perform dressage and other highly disciplined routines.

Preferred "animals" are pets or companion animals and farm animals. Examples of pets are dogs, cats, birds, aquarium fish, guinea pigs, (jack) rabbits, hares and ferrets. Examples of farm animals are aquaculture fish, pigs, horses, ruminants (cattle, sheep and goats) and poultry.

### Nutraceutical uses/formulations/dosages

The term "nutraceutical" as used herein denotes usefulness in both nutritional and pharmaceutical fields of application. Thus, novel nutraceutical compositions can be used as supplements to food and beverages and as pharmaceutical formulations for enteral or parenteral application which may be solid formulations, such as capsules or tablets, or liquid formulations, such as solutions or suspensions.

The nutraceutical compositions according to the present invention may further contain protective hydrocolloids (such as gums, proteins, modified starches), binders, film-forming agents, encapsulating agents/materials, wall/shell materials, matrix compounds, coatings, emulsifiers, surface active agents, solubilising agents (oils, fats, waxes, lecithins etc.), adsorbents, carriers, fillers, co-compounds, dispersing agents, wetting agents, processing aids (solvents), flowing agents, taste-masking agents, weighting agents, jellyfying agents, gel-forming agents, antioxidants and antimicrobials.

Moreover, a multi-vitamin and mineral supplement may be added to nutraceutical compositions of the present invention to obtain an adequate amount of an essential nutrient, which is missing in some diets. The multi-vitamin and mineral supplement may also be useful for disease prevention and protection against nutritional losses and deficiencies due to lifestyle patterns.

The nutraceutical compositions according to the present invention may be in any galenic form that is suitable for administering to the body, especially in any form that is conventional for oral administration, e.g. in solid forms such as (additives/supplements for) food or feed, food or feed premix, fortified food or feed, tablets, pills, granules, dragees, capsules and effervescent formulations, such as powders and tablets, or in liquid forms, such as solutions, emulsions or suspensions as e.g. beverages, pastes and oily suspensions. The pastes may be incorporated in hard- or soft- shell capsules, whereby the capsules feature e.g. a matrix of (fish, swine, poultry, cow) gelatine, plant proteins or ligninsulfonate. Examples for other application forms are those for transdermal, parenteral or injectable administration. The dietary and pharmaceutical compositions may be in the form of controlled (delayed) release formulations.

Examples of food are dairy products including, for example, margarines, spreads, butter, cheese, yoghurts or milk-drinks.

Examples of fortified food are sweet corn, bread, cereal bars, bakery items, such as cakes and cookies, and potato chips or crisps.

Beverages encompass non-alcoholic and alcoholic drinks as well as liquid preparations to be added to drinking water and liquid food. Non-alcoholic drinks are e.g. soft drinks, sports drinks, fruit juices, lemonades, teas and milk-based drinks. Liquid foods are e.g. soups and dairy products. The nutraceutical composition containing *Stevia* extract, its constituents or an enriched *Stevia* extract may be added to a soft drink, an energy bar, or a candy, such that an adult consumes 1 to 1000 mg, more preferably 50-750 mg and most preferably 100-500 mg of *Stevia* extract, its constituents or an enriched *Stevia* extract per daily serving(s).

If the nutraceutical composition is a pharmaceutical formulation the composition further contains pharmaceutically acceptable excipients, diluents or adjuvants. Standard techniques may be used for their formulation, as e.g. disclosed in Remington's Pharmaceutical Sciences, 20th edition Williams & Wilkins, PA, USA. For oral administration, tablets and capsules are preferably used which contain a suitable binding agent, e.g. gelatine or polyvinyl pyrrolidone, a suitable filler, e.g. lactose or starch, a suitable lubricant, e.g. magnesium stearate, and optionally further additives. Preferred are formulations containing 10 to 750 mg, more preferably 50 to 500 mg, of the *Stevia* extract or its constituents, per administration unit, e.g. per tablet or capsule.

For animals including humans a suitable daily dosage of *Stevia* extract or its constituents, for the purposes of the present invention, may be within the range from 0.15 mg per kg body weight to about 10 mg per kg body weight per day. More preferred are nutraceutical compositions of the present invention which contain *Stevia* extract or its constituents, preferably in an amount sufficient to administer to a human adult (weighing about 70 kg) a dosage from about 10 mg/day to about 750 mg/day, preferably from about 50 mg/day to about 500 mg/day.

The following non-limiting Examples are presented to better illustrate the invention.

### Example 1

### Inhibition of glycine transporter 1 in a cellular assay

CHO cells stably expressing the human glycine transporter 1b cDNA (GlyT1) were routinely grown in Dulbecco's Modified Eagle's Medium (Invitrogen, Carlsbad, USA) containing 10 % dialysed foetal calf serum, penicillin, streptomycin, proline and the antibiotic G418. Cells were harvested by trypsinisation one day prior to the assay and were seeded in the above mentioned medium. Immediately prior to the assay, the medium was replaced by uptake buffer containing 150 mM NaCl, 1 mM CaCl₂, 2.5 mM KCl, 2.5 mM MgCl₂, 10 mM Glucose and 10 mM N-2-hydroxyethylpiperazine-N'-2-ethanesulfonic acid (HEPES buffer).

Glycine uptake into the cells was determined by addition of 60 nM radio-labelled [³H]-glycine (Amersham Biosciences GE Healthcare, Slough, UK) and incubation for 30 minutes at room temperature. Following removal of unincorporated label by gentle washing three times with the above buffer, incorporated glycine was quantified by liquid scintillation counting.

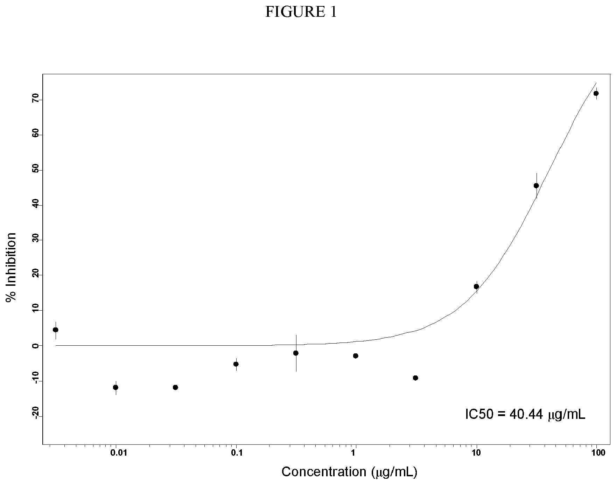

Glycine uptake via the GlyT1 transporter was inhibited by the addition of *Stevia* extract in a dose-dependent manner. Sarcosine, ORG24598 and ALX5407 (all from Sigma, St. Louis, USA) were used as known inhibitors of GlyT1. The measured IC₅₀ values for inhibition of glycine uptake *(Stevia* extract, extract constituents and reference compounds) and representative dose-response curve *(Stevia* extract) are shown in Table 2 and Figure 1, respectively.

Table 2: Measured IC₅₀ values for inhibition of glycine uptake into CHO cells by *Stevia* extract and its major components, steviol, isosteviol and stevioside, in addition to the reference compounds sarcosine, ORG24598 and ALX5407. Data is shown as mean ± s.e.m. (where experiments were performed more than once); IC₅₀ is stated as µg/ml for the extract and µM for pure compounds.

| **Substance** | **IC₅₀ for tritiated glycine uptake** |
|---|---|
| *Stevia* Extract^{∗} | 45 ± 6.9 µg/ml |
| Steviol (Chromadex; Cat. No. ASB-00019352-025) | inactive |
| Isosteviol (Wako Chemicals; Cat. no. 090-02341) | inactive |
| Stevioside (Indofine Chemical Co.; Cat. no. 023718) | inactive |
| Sarcosine (Sigma; Cat. no. S7672) | 35.9 µM |
| ORG24598 (Sigma; Cat. no. O7639) | 0.02 µM |
| ALX5407 (Sigma; Cat. no. A8977) | 6.46 nM |
| Rebaudioside A | inactive |
| *Gingko biloba* (Huisong Pharmaceuticals, GB 102-070116) | inactive |

| | |
|---|---|
| ^{∗} Stevioside: ca. 70 %; Rebaudiosides ca. 20 %; Steviol, Isosteviol; ca. 10 %. | |

The ground leaves of *Stevia rebaudiana* are mixed with hot water for 20-30 min. Subsequently, the aqueous extract is removed by draining, using pressure in order to achieve the maximum amount of extract.

### Example 2

### Hippocampal slice cultures

Seven-day-old Wistar rats were decapitated using a guillotine. In less than 1 minute the skull was opened, the cerebral hemispheres were separated and transferred and both hippocampi were dissected and transferred into ice-cold buffer containing 137 mM NaCl, 5 mM KCl, 0.85 mM Na₂HPO₄, 1.5 mM CaCl₂, 0.66 mM KH₂PO₄, 0.28 mM MgSO₄, 1 mM MgCl₂, 2.7 mM NaHCO₃, 1 mM Kynurenic acid and 0.6 % D-glucose.

Transverse hippocampal slices (400 µm) were prepared using a vibrating blade microtome (VT1200S; Leica Microsystems (Schweiz) AG, Heerbrugg, Switzerland) in the same buffer. Hippocampal slices were individually placed on membrane inserts (Millicell Culture Plate Inserts, 0.4 µm) and cultivated at 35 °C, 5 % CO₂, 95 % humidity in a medium containing a 1: 1 mixture of BME and MEM (both from Invitrogen) containing 25 % heat-inactivated horse serum, 1x GlutaMAX, 1x Penicillin/Streptomycin, 0.6 % glucose and 1 mM Kynurenic acid (Stoppini 1991 J. Neurosci. Methods 37(2): 173-82).

After 48 h in culture, synaptic NMDA receptors were activated by addition of *Stevia* extract, its major constituents or control substances for 15 min in 140 mM NaCl, 5 mM KCl, 1.3 mM CaCl₂, 25 mM HEPES (pH 7.3), 33 mM D-glucose and 0.02 mM bicuculline methiodide. Sarcosine (100 µM) and ALX5407 (20 nM) were used routinely as positive controls. An additional positive control comprised the addition of 200 µM glycine to sister cultures. After the treatments, sections were washed and fixed for immunohistochemistry. Markers of enhanced synaptic activity, normally associated with long-term potentiation, representing an *ex vivo* model of learning and memory were quantitated (see Table 3, below).

Table 3: Relative activation of synaptic markers after treatment with *Stevia* extract or some of its constituent compounds (steviol, isosteviol, stevioside and Rebaudioside A) in comparison with sister cultures treated with buffer. The activation of any of these markers (or a combination thereof) is observed in classical LTP experiments.

| **Substance** | **pCREB** | **pMAPK** | **GluR1** |
|---|---|---|---|
| *Stevia* Extract | ± | + | 255 % |
| Steviol | ++ | ± | 340 % |
| Isosteviol | + | ± | 361 % |
| Stevioside | ± | ++ | ± |
| Rebaudioside A | ± | ± | ± |
| Ginkgo biloba | ± | + | ± |

| | | | |
|---|---|---|---|
| ++++ shows a qualitative maximal activation, ++ and + signify a half-maximum and a moderate activation, respectively, while ± indicates no change in immunoreactivity; for GluR1, values are shown as percentage increase of control values. | | | |

Treatment of hippocampal cultures with *Stevia* extract, steviol, isosteviol and stevioside induced one or more biochemical markers typical for LTP (pCREB: activated form of the cAMP response element binding protein; pMAPK: activated form of the mitogen-activated protein kinase; GluR1: cell surface presence of AMPA receptor 1).

### Example 3

### Preparation of a soft gelatine capsule

A soft gelatine capsule may be prepared comprising the following ingredients:

| **Ingredient** | **Amount per Capsule** |
|---|---|
| *Stevia* extract (freeze-dried/spray -dried) | 200 mg |
| Lecithin | 50 mg |
| Soy bean oil | 250 mg |

Two capsules per day for 3 months may be administered to a human adult. Cognitive function, alertness and the ability to focus on work are seen to improve.

### Example 4

### Preparation of an instant flavoured soft drink

| Ingredient | Amount [g] |
|---|---|
| *Stevia* extract | 0.5 |
| Sucrose, fine powder | 763.1 |
| Ascorbic acid, fine powder | 2.0 |
| Citric acid anhydrous powder | 55.0 |
| Lemon flavour | 8.0 |
| Trisodium citrate anhydrous powder | 6.0 |
| Tricalciumphosphate | 5.0 |
| β-Carotene 1% CWS from DNP AG, Kaiseraugst, Switzerland | 0.4 |
| Total amount | 840 |

All ingredients are blended and sieved through a 500µm sieve. The resulting powder is put in an appropriate container and mixed in a tubular blender for at least 20 minutes. For preparing the drink, 105g of the obtained mixed powder are mixed with sufficient water to produce one litre of beverage.

The ready-to-drink soft drink contains ca. 15 mg enriched *Stevia* extract per serving (250 ml). As a strengthener and for general well-being 2 servings per day (500ml) may be drunk. Cognitive function, alertness and the ability to focus on work are seen to improve.

### Example 5

### Preparation of a fortified non-baked cereal bar

| Ingredient | Amount [g] |
|---|---|
| *Stevia* extract | 0.3 |
| Water | 54.0 |
| Salt | 1.5 |
| Glucose syrup | 130.0 |
| Invert sugar syrup | 95.0 |
| Sorbitol Syrup | 35.0 |
| Palm kernel fat | 60.0 |
| Baking fat | 40.0 |
| Lecithin | 1.7 |
| Hardened palm-oil | 2.5 |
| Dried and cut apple | 63.0 |
| Cornflakes | 100.0 |
| Rice crispies | 120.0 |
| Wheat crispies | 90.0 |
| Roasted hazelnut | 40.0 |
| Skimmed milk powder | 45.0 |
| Apple flavour 74863-33 | 2.0 |
| Citric acid | 5.0 |
| Total amount | 885 |

The enriched *Stevia* extract is premixed with skimmed milk powder and placed in a planetary bowl mixer. Cornflakes and rice crispies are added and the total is mixed gently. Then the dried and cut apples are added. In one cooking pot, water and salt are mixed in the amounts given above (solution 1). In a second cooking pot, glucose-, invert sugar- and sorbitol- syrups are mixed in the amounts given above (solution 2). The fat phase constitutes a mixture of baking fat, palm kernel fat, lecithin and emulsifier. Solution 1 is heated to 110°C. Solution 2 is heated to 113°C and then cooled in a cold water bath.

Subsequently, solutions 1 and 2 are combined. The fat phase is melted at 75°C in a water bath, then added to the combined mixture of solutions 1 and 2. Apple flavour and citric acid are added to the liquid sugar/fat mix. The liquid mass is added to the dry ingredients and mixed well in the planetary bowl mixer. The mass is put on a marble plate and rolled to the desired thickness. The mass is cooled down to room temperature and cut into pieces. The non-baked cereal bar contains ca. 10 mg enriched *Stevia* extract per serving (30 g). For general well-being and energising 1-2 cereal bars may be eaten per day. Cognitive function, alertness and the ability to focus on work are seen to improve.

### Example 6

### Dry dog feed containing Stevia extract

A commercial basal diet for dogs (e.g. Mera Dog "Brocken", MERA-Tiernahrung GmbH, Marienstraße 80-84, D-47625 Kevelaer-Wetten, Germany) is sprayed with a solution of *Stevia* extract in water, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to a dog a daily dose of 4 mg *Stevia* extract per kg body weight. The food composition is dried to contain dry matter of about 90% by weight. For an average dog of 10 kg body weight to consume approx. 200 g dry feed per day, the dog food contains approx. 200 mg *Stevia* extract per kg food. For heavier dogs, the feed mix is prepared accordingly. For reduction of stress, fear and aggressiveness in dogs, the food can be given to dogs in animal shelter farms on a regular basis. Before veterinarian visits or stays in veterinarian clinics or holiday separation, the food is given at least one week before, during the stressful event and one week thereafter.

### Example 7

### Wet cat food containing Stevia extract

A commercial basal diet for cats (e.g. Happy Cat "Adult", Tierfeinnahrung, Südliche Hauptstraße 38, D-86517 Wehringen, Germany) is mixed with a solution of *Stevia* extract in water, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to a cat a daily dose of 4 mg *Stevia* extract per kg body weight. For an average cat of 5 kg of body weight to consume approx. 400 g of wet food, the cat food contains 50 mg *Stevia* extract per kg food. The food composition is dried to contain dry matter of about 90% by weight. For reduction of stress, fear and aggressiveness in cats, the food can be given to cats in animal shelter farms on a regular basis. Before veterinarian visits or stays in veterinarian clinics, the food is given at least one week before, during the stressful event, and one week thereafter.

### Example 8

### Dog treats containing Stevia extract

Commercial dog treats (e.g. Mera Dog "Biscuit" for dogs as supplied by Mera Tiernahrung GmbH, Marienstrasse 80-84, 47625 Kevelaer-Wetten, Germany) are sprayed with a solution of *Stevia* extract in water, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and monophosphate esters of sodium/calcium L-ascorbate (e.g. ROVIMIX® STAY-C® 35 from DSM Nutritional Products Ltd, Kaiseraugst, Switzerland) in an amount sufficient to administer to the treats 0.5 - 5 mg *Stevia* extract per g treats. The food composition is dried to contain dry matter of about 90% by weight. To reduce fear and tension, the treat can be given during the day in addition to the food, or when feeding is not warranted, i.e. while travelling, for up to 5 times per day.

### Example 9

### Cat treats containing Stevia extract

Commercial cat treats (e.g. Whiskas Dentabits for cats as supplied by Whiskas, Masterfoods GmbH, Eitzer Str. 215, 27283 Verden/Aller, Germany) are sprayed with a solution of *Stevia* extract in water, together with antioxidants such as vitamin C (e.g. ROVIMIX® C-EC from DSM Nutritional Products Ltd, Kaiseraugst, Switzerland) and its derivatives, i.e. sodium ascorbyl monophosphate (e.g. STAY-C® 50 from DSM Nutritional Products Ltd, Kaiseraugst, Switzerland) or a mixture of tri-, di- and mono- phosphate esters of sodium/calcium L-ascorbate (e.g. ROVIMIX® STAY-C® 35 from DSM Nutritional Products Ltd, Kaiseraugst, Switzerland) in an amount sufficient to administer to the treats 0.5 - 5 mg *Stevia* extract per g treats. The food composition is dried to contain dry matter of about 90% by weight. To reduce fear and tension, the treat can be given during the day in addition to the food, or when feeding is not warranted, i.e. while travelling, for up to 5 times per day.

## Claims

1. The non-therapeutic use of steviol in improving cognitive function which is selected from the group consisting of: maintaining cognitive wellness and balance, learning, language processing, problem solving, intellectual functioning, ability to cope with psychosocial burdens, attention and concentration, memory, the capacity for remembering, mental alertness, mental vigilance, mental fatigue, stabilisation of mental status, a stress reliever, work overload stress, stress-related exhaustion and/or burn out, and to promote relaxation in an animal or human.

2. Non-therapeutic use of steviol according to claim 1 which is for veterinary use.

## Patentansprüche

1. Nichttherapeutische Verwendung von Steviol beim Verbessern der kognitiven Funktion ausgewählt aus der Gruppe bestehend aus: Aufrechterhaltung von kognitivem Wohlbefinden und Gleichgewicht, Lernen, Sprachverarbeitung, Problemlösen, intellektueller Funktion, Fähigkeit zum Bewältigen von psychosozialen Belastungen, Aufmerksamkeit und Konzentration, Gedächtnis, Erinnerungsfähigkeit, mentaler Aufgewecktheit, mentaler Wachsamkeit, mentaler Ermüdung, Stabilisierung des mentalen Status, Stressabbau, Arbeitsüberlastungsstress, stressbedingter Erschöpfung und/oder Burnout und zur Förderung von Entspannung bei einem Tier oder einem Menschen.

2. Nichttherapeutische Verwendung von Steviol nach Anspruch 1 für die tierärztliche Verwendung.

## Revendications

1. Utilisation non thérapeutique de stéviol dans l'amélioration de la fonction cognitive qui est choisie dans le groupe constitué par : le maintien du bien-être et de l'équilibre cognitifs, l'apprentissage, le traitement du langage, la résolution de problèmes, le fonctionnement intellectuel, la capacité à faire face à des charges psychologiques, l'attention et la concentration, la mémoire, la capacité de remémoration, la vivacité d'esprit, la vigilance mentale, la fatigue mentale, la stabilisation de l'état mental, un soulagement de stress, un stress de surcharge de travail, un épuisement et/ou un burn-out liés au stress, et pour favoriser la relaxation chez un animal ou un humain.

2. Utilisation non thérapeutique de stéviol selon la revendication 1 qui est pour une utilisation vétérinaire.
